**Europäisches Patentamt**

(19) **European Patent Office**

**Office européen des brevets**

(11) Veröffentlichungsnummer: **0 219 568 B1**

(12) **EUROPÄISCHE PATENTSCHRIFT**

(45) Veröffentlichungstag der Patentschrift:
**11.10.89**

(51) Int. Cl.⁴: **A61B 17/39**

(21) Anmeldenummer: **85113437.9**

(22) Anmeldetag: **23.10.85**

(54) **Hochfrequenz-Chirurgiegerät.**

(43) Veröffentlichungstag der Anmeldung:
**29.04.87 Patentblatt 87/18**

(45) Bekanntmachung des Hinweises auf die Patenterteilung:
**11.10.89 Patentblatt 89/41**

(84) Benannte Vertragsstaaten:
**DE FR GB IT**

(56) Entgegenhaltungen:
**DE-C- 2 801 833**

(73) Patentinhaber: **Erbe Elektromedizin GmbH.,
Ebertstrasse 35, D-7400 Tübingen(DE)**

(72) Erfinder: **Farin, Günter, Kapellenweg 15,
D-7400 Tübingen(DE)**
Erfinder: **Pütz, Peter, Weissdornweg 14/116,
D-7400 Tübingen(DE)**
Erfinder: **Haag, Reiner, Käsbachstrasse 7,
D-7403 Pfäffingen(DE)**

(74) Vertreter: **Endlich, Fritz, Dipl.-Phys.,
Postfach 1326 Blumenstrasse 8, D-8034 Germering(DE)**

## Beschreibung

Die Erfindung betrifft ein Hochfrequenz-Chirurgiegerät zum Schneiden und/oder Koagulieren biologischen Gewebes mit automatischer Kontrolle oder Regelung des Hochfrequenz-Stromes während des Schneidens und/oder während der Koagulation wobei als Kriterium für die Kontrolle oder Regelung die Intensität des elektrischen Lichtbogens zwischen der aktiven Elektrode und dem zu schneidenden und/oder zu koagulierenden Gewebe genutzt wird.

Die Erfindung geht von der Erkenntnis aus, daß die zum Schneiden biologischer Gewebe erforderliche hohe Dichte des elektrischen Stromes an der Stelle des Gewebes, wo der Schnitt erfolgen soll, in der Regel nur dann erreicht wird, wenn die sogenannte aktive Elektrode das Gewebe nicht direkt elektrisch leitfähig berührt, sondern wenn bei ausreichend hoher elektrischer Spannung zwischen Gewebe und aktiver Elektrode ein elektrischer Lichtbogen entsteht, der den gesamten elektrischen Strom punktförmig auf die zu schneidende Stelle des Gewebes konzentriert.

Diese Erkenntnis ist nicht neu sondern nur weitgehend in Vergessenheit geraten. 1909 wurde von DE FOREST erstmals eine Schneidewirkung durch den von einer Elektrode auf das Gewebe überspringenden elektrischen Funken erzielt. Der sogenannte Funkenschnitt nach DE FOREST wurde in Deutschland ab 1910 von M. COHN und von CZERNY eingeführt. J. KOWARSCHIK (1928) und H.v. SEEMEN (1932), beide Verlag J. Springer, Berlin, beschreiben den sog. Funkenschnitt noch ausführlich. Neuere Autoren dagegen beschreiben als Ursache der Schneidewirkung die hohe Stromdichte des elektrischen Stromes, welche bei kleinflächigem direktem Kontakt zwischen der aktiven Elektrode und dem zu schneidenden Gewebe erreicht wird, ohne eine elektrischen Lichtbogen oder Funken kausal zu erwähnen. In der DE 31 19 735 A1 wird ein "Verfahren und eine Schaltungsanordnung zur Regelung der Ausgangsleistung eines Elektrochirurgie-Hochfrequenz-Generators" beschrieben, mittels welcher "an der Schneideelektrode die Entladestromdichte auf einen geringeren als zur Zündung eines Lichtbogens notwendigen Wert beschränkt bleibt".

In der Deutschen Auslegeschrift 28 01 833 B1 geht der Erfinder von der Hypothese aus, daß die Schneidewirkung von der Temperatur der Schneideelektrode abhängig ist, so daß es Aufgabe seiner Erfindung ist, die Temperatur der Schneideelektrode während des Schneidens in einem gewünschten Temperaturbereich zu halten. Als Kriterium für die Temperatur führt der Erfinder eine Gleichspannung an, welche sich proportional zur Temperatur der Schneideelektrode zwischen der Schneideelektrode und dem Gewebe ausbildet und welche es zu regeln gilt, um damit indirekt die Temperatur der Schneideelektrode in dem gewünschten Temperaturbereich zu halten.

Sowohl in der DE 31 19 735 A1 als auch in der DAS 28 01 833 B1 wird die kausale Bedeutung des elektrischen Lichtbogens für die Schneidewirkung des elektrischen Stromes nicht richtig erkannt, so daß die hierin vorgeschlagenen Verfahren zur Regelung der Ausgangsleistung der Hochfrequenzgeneratoren für hochfrequenz-chirurgische Schnitte von falschen Hypothesen ausgehen und folglich nicht realisierbar sind.

Aus dem DP 25 04 280 ist eine Vorrichtung zum Schneiden und/oder Koagulieren menschlichen Gewebes mit Hochfrequenzstrom bekannt, welche von der kausalen Bedeutung des elektrischen Lichtbogens zwischen der aktiven Elektrode, dort Sonde genannt, und dem zu schneidenden oder zu koagulierenden Gewebe ausgeht. Diese Vorrichtung ist dadurch gekennzeichnet, daß eine Anzeigeeinrichtung das Ausmaß eines sich zwischen der Sonde und dem Gewebe ausbildenden Lichtbogens durch ein elektrisches Signal anzeigt, und daß der Regeleinrichtung das elektrische Signal der Anzeigeeinrichtung und ein Sollwertprogramm aus einem Sollwertgeber zugeführt ist und die Regeleinrichtung aus diesen beiden Daten die Regelgröße derart ableitet und dem Generator zuführt, daß die Stromstärke des Hochfrequenzstromes jeweils auf einen dem Sollwertprogramm und dem gewünschten Ausmaß des Lichtbogens entsprechenden Wert eingestellt ist.

Zur Gewinnung eines für die Regelung geeigneten elektrischen Signals wird in der DP 25 04 280 vorgeschlagen, den Einsatzpunkt und die Stärke des Lichtbogens mit Hilfe der Stärke der in dem der Sonde zugeführten Hochfrequenzstrom enthaltenen Ströme der harmonischen Frequenzen der Betriebsfrequenz zu analysieren.

Da Hochfrequenz-Leistungsgeneratoren mit Rücksicht auf einen möglichst hohen Wirkungsgrad in der Regel außer der Grundfrequenz auch harmonische Frequenzen der Grundfrequenz erzeugen, deren Pegel mit wachsendem Wirkungsgrad zunimmt, schlägt die DP 25 04 280 vor, daß die Anzeigeeinrichtung ein Filter enthält, das zwischen Generator und Sonde liegt und Ströme der Betriebsfrequenz (Synonym für Grundfrequenz) durchläßt und Ströme der harmonischen Frequenzen sperrt und die Messung der Ströme der harmonischen Frequenzen an dem dem Generator abgewandten Ausgangstor des Filters erfolgt. Durch die Einfügung eines Tiefpaßfilters in den Ausgangskreis des Hochfrequenzgenerators muß allerdings ein geringerer Wirkungsgrad des Hochfrequenzgenerators in Kauf genommen werden.

Bei der praktischen Realisation eines Hochfrequenz-Chirurgiegerätes entsprechend des DP 25 04 280 hat es sich erwiesen, daß der Filteraufwand zur Unterdrückung der Ströme der harmonischen Frequenzen konventioneller Hochfrequenzgeneratoren relativ hoch ist und der Wirkungsgrad relativ gering wird.

Um die beiden Anforderungen an den Hochfrequenzgenerator für die Vorrichtung zum Schneiden und/oder Koagulieren entsprechend der DP 25 04 280, nämlich möglichst geringer Anteil Ströme harmonischer Frequenzen der Grundfrequenz einerseits und möglichst hoher Wirkungsgrad andererseits, zu realisieren, wird in dem ersten serienmäßig hergestellten Hochfrequenz-Chirurgiegerät entsprechend des DP 25 04 280 ein spezieller, selektiver Verstärker entsprechend der DP 32 27 109.3 verwendet. Dieser selektive Verstärker enthält jedoch ebenfalls aufwendige und teuere selektive Resonanzkreise und Filter, welche außerdem relativ genau auf die Grundfrequenz bzw. deren harmonische Frequenzen abgestimmt sein müssen.

Es ist daher Aufgabe der Erfindung, ein Hochfrequenz-Chirurgiegerät mit automatischer Regelung der Intensität des elektrischen Lichtbogens zwischen der aktiven Elektrode und dem zu schneidenden und/oder zu koagulierenden Gewebe zu entwickeln, welches im Vergleich zu dem bekannten Hochfrequenz-Chirurgiegerät entsprechend dem DP 25 04 280 weniger Aufwand an Filtern und Filterabgleicharbeiten erfordert und auch ohne spezielle, selektive Leistungsverstärker entsprechend DP 32 27 109.3, welche ebenfalls zusätzliche aufwendige selektive Bauteile im Vergleich zu bekannten Leistungsverstärkern erfordern, betrieben werden kann.

Umfangreiche Untersuchungen über die Vorgänge an der aktiven Elektrode während des Schneidens und/oder Koagulierens führten zu der Erkenntnis, daß der für das Schneiden biologischer Gewebe erforderliche elektrische Lichtbogen zwischen der aktiven Elektrode und dem Gewebe sowohl zeitlich als auch örtlich sehr variabel ist. Sobald die elektrische Feldstärke an einer oder mehreren Stellen zwischen aktiver Elektrode und Gewebe ausreichend hoch ist, zündet an dieser bzw. an diesen Stellen ein bzw. mehrere elektrische Lichtbogen. An der Stelle, an welcher der jeweilige elektrische Lichtbogen das biologische Gewebe berührt, wird das Gewebe lokal sehr schnell so stark erhitzt, daß der Wassergehalt und andere verdampfbare Bestandteile des Gewebes an dieser Stelle sofort verdampfen, wodurch der betroffene Gewebeteil sofort nach der Berührung mit dem elektrischen Lichtbogen verschwindet und an seiner Stelle ein partieller Krater entsteht. Hierdurch wird an dieser partiellen Stelle der Abstand zwischen der aktiven Elektrode und dem biologischen Gewebe größer und dadurch die elektrische Feldstärke eben an dieser partiellen Stelle entsprechend geringer, so daß der elektrische Lichtbogen an dieser partiellen Stelle entweder erlischt oder zum Rand des entstandenen Kraters ausweicht oder an einer anderen partiellen Stelle zwischen aktiver Elektrode und biologischem Gewebe zündet, nämlich da, wo die nächst günstige Zündbedingung besteht. Sobald ein elektrischer Lichtbogen an einer beliebigen partiellen Stelle zwischen der aktiven Elektrode und dem biologischen Gewebe gezündet hat, untergräbt er innerhalb kürzester Zeit seine Existenzgrundlage an der Stelle des biologischen Gewebes, an der er zündete, so daß er auf andere Stellen ausweicht oder an anderen Stellen neu zünden muß. Dieser Vorgang läuft stochastisch und mit sehr hoher Geschwindigkeit ab. Die Summe aller so entstehenden partiellen Krater bilden den Schnitt bzw. den Schneidespalt im biologischen Gewebe.

Aufgrund dieser stochastisch und mit hoher Geschwindigkeit zündenden und erlöschenden elektrischen Lichtbogen zwischen aktiver Elektrode und biologischem Gewebe verhält sich die Strecke zwischen aktiver Elektrode und biologischem Gewebe wie ein elektrischer Widerstand mit starkem, breitbandigem Rauschen. Modellhaft kann diese Strecke hier auch als Rauschquelle betrachtet werden, deren Rauschleistung der Intensität der elektrischen Lichtbogen proportional ist. Die Proportionalität zwischen Rauschleistung und Intensität der elektrischen Lichtbogen ist ausreichend gut, um diese als Kriterium für die Regelung der Intensität der Lichtbogen zu nutzen.

Da der Lichtbogen zwischen aktiver Elektride und biologischem Gewebe, wie seit langem bekannt, infolge seines nicht linearen Verhaltens als elektrischer Widerstand bezüglich Proportionalität zwischen Strom und Spannung außerdem mehr oder weniger Gleichrichtereffekt hat, reicht das Frequenzspektrum des Rauschens von der Frequenz Null bis weit oberhalb der Grundfrequenz des Hochfrequenzgenerators.

Bei der Verwendung einer selektiven Frequenz oder eines mehr oder weniger breiten Frequenzbandes aus dem sehr breiten Frequenzspektrum des Rauschens ist es mit Rücksicht auf eine möglichst hohe Regelgeschwindigkeit zweckmäßig, möglichst hohe Frequenzen aus diesem Spektrum zu nutzen, wobei jedoch mit Rücksicht auf die Abnahme der Amplitude nur Frequenzen unterhalb etwa $f_6$ von praktischem Interesse sind. Insbesondere ist die harmonische Frequenz fo (gleich 0 Hz) nicht für eine Regelung geeignet, weil die Regelzeitkonstante hierbei unendlich wäre. Es ist jedoch unbedingt darauf zu achten, daß die Grundfrequenz und die harmonischen Frequenzen der Grundfrequenz, die vom Hochfrequenzgenerator des Hochfrequenz-Chirurgiegerätes geliefert werden, für die Regelung nicht geeignet sind. Geeignet dagegen sind mehr oder weniger gut alle anderen Frequenzen des durch den elektrischen Lichtbogen verursachten Rauschens.

Für die Gewinnung eines der Intensität des oder der elektrischen Lichtbogen zwischen der aktiven Elektrode und dem Gewebe ausreichend proportionalen elektrischen Signales ist ein elektrisches Filter erforderlich, das entweder eine selektive Frequenz, außer der Grundfrequenz oder der vom Hochfrequenzgenerator gelieferten harmonischen Frequenz der Grundfrequenz, oder mehrere Frequenzen oder mehr oder weniger breite Frequenzbänder, exklusive der Grundfrequenz und den vom Hochfrequenzgenerator gelieferten harmonischen Frequenzen der Grundfrequenz, aus dem vom elektrischen Lichtbogen verursachten elektrischen Rauschen herauszufiltern und elektrisch für die Regelung aufzubereiten.

Die nichtharmonischen Frequenzen können prinzipiell sowohl aus der elektrischen Spannung zwischen aktiver Elektrode und Gewebe als auch aus dem elektrischen Strom der durch die aktive Elektrode in das Gewebe fließt, herausgefiltert werden. Hierbei ist unbedingt darauf zu achten, daß die Intensitäten niederfrequenter Ströme durch das Gewebe unterhalb der elektrischen Reizschwellen von Nerven- und Muskelzellen bleiben müssen.

Besonders vorteilhaft ist diese Lösung im Vergleich zu dem bekannten Hochfrequenz-Chirurgiegerät entsprechend dem DP 25 04 280 insofern, als der Leistungsverstärker des erfindungsgemäßen Hochfrequenz-Chirurgiegerätes weder selektiv bzw. frei von harmonischen Frequenzen der Grundfrequenz noch besonders frequenzstabil sein muß. Vorteilhaft ist diese Lösung weiterhin insofern, da nur ein Filter zur Gewinnung eines für die Regelung geeigneten Signales erforderlich ist, das keine große Leistung übertragen muß und daher relativ klein und kostengünstig hergestellt werden kann.

Während zum Schneiden elektrische Lichtbogen zwischen der aktiven Elektrode und dem Gewebe, wie oben beschrieben, erforderliche sind, müssen diese während des Koagulierens möglichst vermieden werden.

Für Schneidevorgänge sind daher Sollwertgeber erforderlich, welche die Amplitude des Ausgangssignales des Leistungsverstärkers des Hochfrequenz-Chirurgiegerätes so hoch steuern, daß stets die zum Schneiden erforderlichen elektrischen Lichtbogen zwischen der aktiven Elektrode und dem Gewebe vorhanden sind.

Für Koagulationsvorgänge sind dagegen Sollwertgeber erforderlich, welche die Amplitude des Ausgangssignales des Leistungsverstärkers des Hochfrequenz-Chirurgiegerätes entweder so steuert, daß zwischen der aktiven Elektrode und dem Gewebe keine elektrischen Lichtbogen entstehen können oder aber daß nur soviel Lichtbogen entstehen, daß praktisch noch kein Schnitt erfolgt.

Für koagulierende Schneidevorgänge, bei welchen die Schnittflächen während des Schneidevorganges gleichzeitig mehr oder weniger stark koaguliert werden sollen, sind Sollwertgeber erforderlich, welche in ausreichend schneller Folge die Amplitude des Ausgangssignales des Leistungsverstärkers des Hochfrequenz-Chirurgiegerätes so steuern, daß Koagulationsintervalle und Schneideintervalle einander ablösen. Derartige Sollwertgeber sind aus der DP 35 30 335.2 bekannt.

Anhand der Zeichnungen Figur 1 bis Figur 5 soll die Erfindung im folgenden detaillierter beschrieben werden.

Es zeigen:

Fig. 1 eine schematische Darstellung des Frequenzspektrums eines Hochfrequenzgenerators von einem üblichen bekannten Hochfrequenz-Chirurgiegerät, während zwischen der aktiven Elektrode und dem Gewebe kein elektrischer Lichtbogen vorhanden ist.

Fig.2 + 3 eine schematische Darstellung des Frequenzspektrums des Stromes oder der Spannung zwischen aktiver Elektrode und Gewebe während elektrische Lichtbogen vorhanden sind.

Fig. 4 die Kriterien für die Gestaltung des Hochfrequenzfilters.

Fig. 5 ein Auführungsbeispiel des erfindungsgemäßen Hochfrequenz-Chirurgiegerätes.

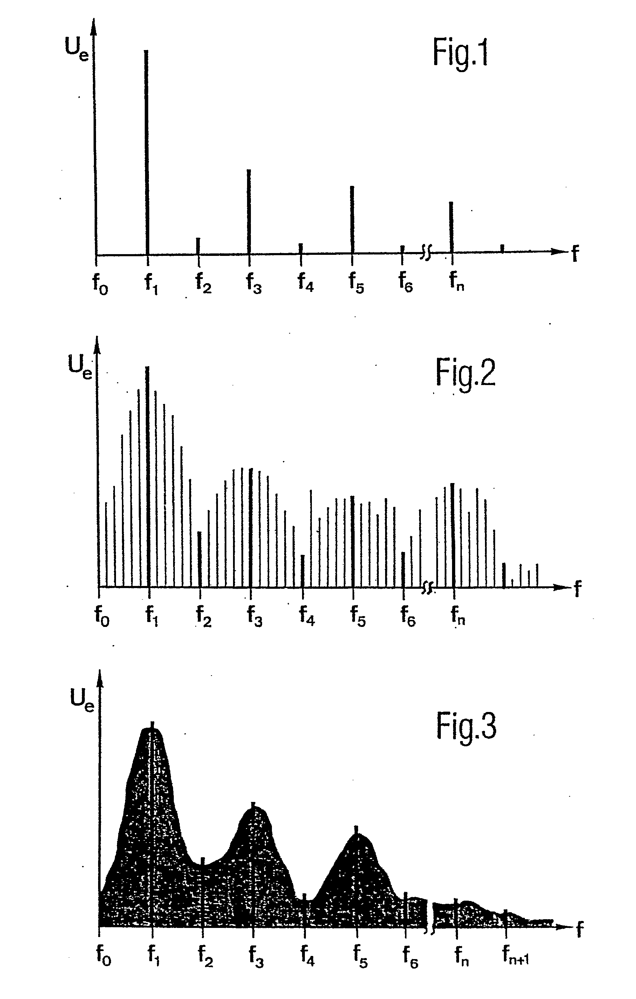

Figur 1 zeigt ein typisches Frequenzspektrum bekannter Hochfrequenzgeneratoren für die Hochfrequenzchirurgie. Da diese Hochfrequenzgeneratoren zur Erzielung eines hohen Wirkungsgrades mit Rücksicht auf die Verlustleistung innerhalb des Hochfrequenzgenerators mit Gegentaktverstärkern in B- oder C- oder gar D-Betrieb verwendet werden, liefern diese Generatoren außer der Grundfrequenz $f_1$ auch mehr oder weniger intensiv die ganzzahligen Vielfachen der Grundfrequenz $f_1$, die sog. harmonischen Frequenzen $f_2$, $f_3$, $f_4$, $f_5$, $f_6$ .... $f_n$ usw., wobei die ungeradezahligen Harmonischen $f_3$, $f_5$ usw. in der Regel intensiver vorhanden sind als die geradezahligen Harmonischen $f_2$, $f_4$ usw.

Solange zwischen der aktiven Elektrode und dem Gewebe kein elektrischer Lichtbogen vorhanden ist, sind am Ausgang des Hochfrequenzgenerators nur die Grundfrequenz $f_1$ sowie mehr oder weniger intensiv die ganzzahligen harmonischen Frequenzen der Grundfrequenz vorhanden. Die nullte harmonische Frequenz $f_0$ ist hierbei nicht vorhanden.

Sobald jedoch zwischen der aktiven Elektrode und dem Gewebe elektrische Lichtbogen vorhanden sind, entstehen infolge des nichtlinearen Verhaltens des elektrischen Widerstandes der Lichtbogen und infolge der stochastischen Häufigkeit der Lichtbogen pro Zeiteinheit auch nichtharmonische Frequenzen zwischen den harmonischen Frequenzen, wie in Figur 2 schematisch dargestellt. Da die von den Lichtbogen verursachten nichtharmonischen Frequenzen stochastisch erscheinen, ist deren statistische Verteilung, wie in Figur 3 schematisch dargestellt, lückenlos.

Als Kriterium für die automatische Regelung der Intensität des elektrischen Lichtbogens zwischen der aktiven Elektrode und dem Gewebe wird erfindungsgemäß selektiv eine bestimmte nichtharmonische Frequenz oder ein mehr oder weniger breites Frequenzspektrum zwischen zwei benachbarten harmonischen Frequenzen oder mehrere mehr oder weniger breite Frequenzspektren der nichtharmonischen Frequenzen zwischen verschiedenen benachbarten harmonischen Frequenzen durch geeignete Hochfrequenzfilter aus dem in Figur 2 oder Figur 3 dargestellten Gesamtspektrum herausgefiltert und als Regelsignal aufbereitet.

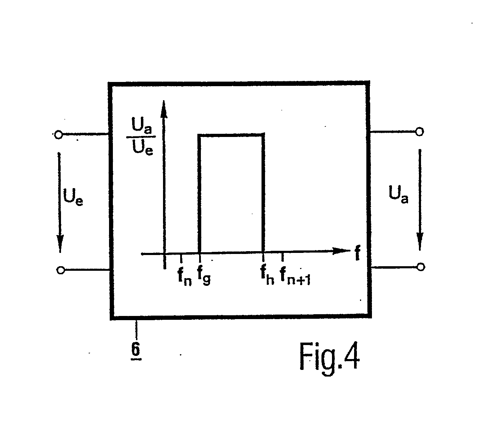

Figur 4 zeigt die Kriterien, die bei der Wahl der für die automatische Regelung der Intensität der elektrischen Lichtbogen zwischen aktiver Elektrode und Gewebe zu beachten sind. Die harmonischen Frequenzen $f_n$ und $f_{n+1}$, wobei n jede ganze Zahl von Null an sein kann, sind für die automatische Regelung nicht geeignet, wenn der Hochfrequenzgenerator des betreffenden Hochfrequenz-Chirurgiegerätes diese Frequenzen mit nicht vernachlässigbarem Pegel liefert. Da die Grundfrequenz $f_1$ der Hochfrequenzgeneratoren von Hochfrequenz-Chirurgiegeräten mit Rücksicht auf die Kosten nicht genau auf eine genormte Frequenz festgelegt ist, die jeweils verwendete Grundfrequenz nicht sehr stabil ist und infolge Amplitudenmodulation durch Netzbrumm und automatischer Regelung Seitenfrequenzbänder entstehen, ist zu beachten, daß nur solche nichtharmonische Frequenzen aus dem jeweiligen Frequenzintervall $f_n$ bis $f_{n+1}$ für die Erzeugung des automatischen Regelsignales verwendet werden, die ausreichenden Abstand von den harmonischen Frequenzen inklusive deren Toleranzen und Seitenfrequenzen haben. In Figur 4 sind die beiden Sicherheitsabstände $f_g$ - $f_n$ und $f_{n+1}$ - $f_h$ schematisch dargestellt. Bei der Wahl der Bandbreite $f_h$ - $f_g$ ist es mit Rücksicht auf die stochastische Wahrscheinlichkeit der durch die elektrischen Lichtbogen erzeugten nichtharmonischen Frequenzen zweckmäßig, diese so breit wie praktisch realisierbar zu wählen, wobei die Flankensteilheit der Durchlaßfunktion $U_a/U_e$ des verwendeten Bandpaßfilters an den Stellen $f_g$ und $f_h$ möglichst groß sein sollte.

Die in Figur 4 dargestellte Durchlaßfunktion $U_a/U_e$ über f ist idealisiert. Bei der Wahl und Dimensionierung dieses Filters sollte versucht werden, dieser idealisierten Durchlaßfunktion möglichst nahe zu kommen. Dem Fachmann sind heute viele verschiedene Hochfrequenzfilter-Schaltungen bekannt, beispielsweise piezokeramische Filter (z.B. Cat. No. p42E-3,1984 der Firma MURATA Mfg. Co. Ltd.).

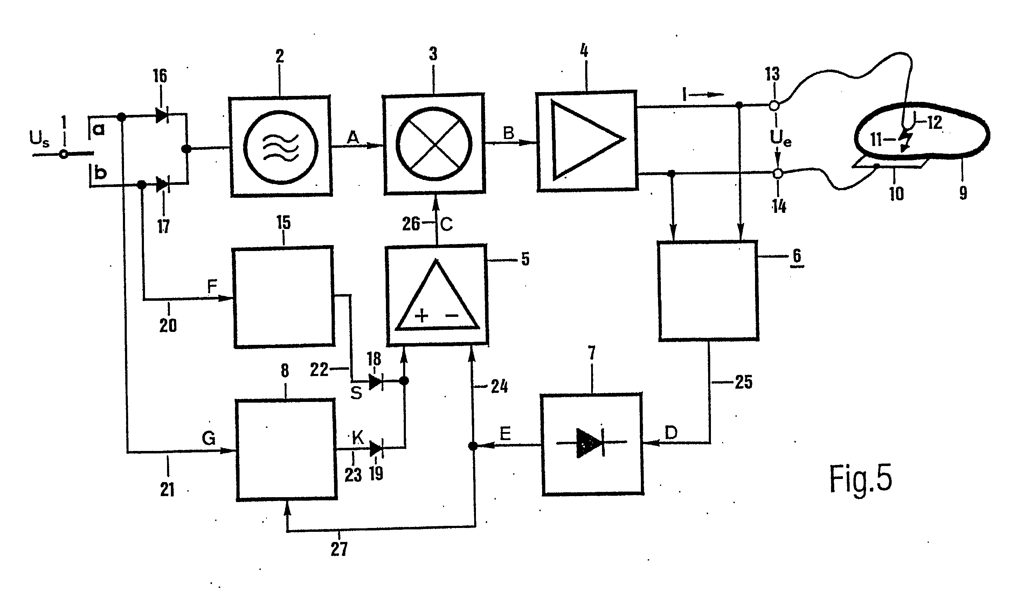

Figur 5 zeigt die erfindungsrelevanten Details eines erfindungsgemäßen Hochfrequenz-Chirurgiegerätes, bestehend aus einem Hochfrequenz-Oszillator 2, einem Amplitudenmodulator 3, einem Leistungsverstärker 5, dem Filter 6 für die nicht harmonischen Frequenzen entsprechend Figur 4, wo dessen Eigenschaften näher beschrieben wurden, dem Gleichrichter 7, dem Sollwertgeber 8 zum Koagulieren , dem Sollwertgeber 15 zum Schneiden, und dem Tastschalter 1 zum Aktivieren des Hochfrequenzgenerators entweder zum Koagulieren, wenn Kontakt a geschlossen ist, oder zum Schneiden, wenn Kontakt b geschlossen ist.

An den Ausgängen 13, 14 des Hochfrequenz-Chirurgiegerätes sind die aktive Elektrode 12 und die Neutralelektrode 10 angeschlossen. Die Neutralelektrode 10 ist an das Gewebe 9 des Patienten elektrisch leitfähig angelegt.

Wird der Tastschalter 1 in Schaltstellung a geschaltet, so wird hierdurch über die Diode 16 der Hochfrequenz-Oszillator 2 aktiviert und gleichzeitig über die Leitung 21 der Sollwertgeber 8 für die Intensität der Koagulationsleistung eingeschaltet. Das Sollwertsignal K für die Koagulationsleistung wird über die Leitung 23 und die Diode 19 dem Regelverstärker 5 zugeführt. Solange zwischen der aktiven Elektrode 12 und dem Gewebe 9, die miteinander in Kontakt sind, kein elektrischer Lichtbogen zündet, wird die Ausgangsleistung des Leistungsverstärkers 4 vom Sollwertgeber 8 gesteuert. Sobald zwischen der aktiven Elektrode 12 und dem Gewebe 9 elektrische Lichtbogen 11 zünden und hierdurch nichtharmonische Frequenzen im Hochfrequenz-Ausgangskreis 13, 14 erscheinen, werden die für die Regelung der Intensität der elektrischen Lichtbogen 11 geeigneten nichtharmonischen Frequenzen durch das Filter 6 herausgefiltert, dem Gleichrichter 7 über die Leitung 25 zugeführt, dort gleichgerichtet und über die Leitung 24 dem Regelverstärker 5 zugeführt. Da das Ausgangssignal E des Gleichrichters 7 weitgehend proportional der Intensität der Lichtbogen 11 ist, wird die Ausgangsleistung des Leistungsverstärkers 4 durch das Ausgangssignal C des Regelverstärkers 5 mittels des Amplitudenmodulators 3 so gesteuert, daß die Intensität der Lichtbogen 11 auf den Sollwert des Sollwertgebers 8 geregelt wird. Das Ausgangssignal E des Gleichrichters 7 wird über die Leitung 27 gleichzeitig dem Sollwertgeber 8 zugeführt, wodurch die Sollwerte für Koagulationen dynamisch so geführt werden können, daß die Ausgangsleistung des Leistungsverstärkers 4 nach dem Zünden der elektrischen Lichtbogen 11 mehr oder weniger schnell so weit abgesenkt wird, daß die elektrischen Lichtbogen 11 wieder erlöschen, wonach die Ausgangsleistung des Leistungsverstärkers 4 mehr oder weniger schnell wieder durch den Sollwertgeber 8 hochgesteuert wird bis wieder elektrische Lichtbogen 11 zünden und diese Zyklen sich so lange wiederholen, wie der Tastschalter 1 in Schaltstellung a geschaltet bleibt. Hierfür geeignete Sollwertgeber sind in dem DP 25 04 280 und in dem DP 35 30 335.2 beschrieben.

Wird der Tastschalter 1 in Schaltstellung b geschaltet, so wird hierdurch über die Diode 17 der Hochfrequenz-Oszillator 2 aktiviert und gleichzeitig über die Leitung 20 der Sollwertgeber 15 für die Intensität der Schneideleistung eingeschaltet. Das Sollwertsignal S für die Schneideleistung wird über die Leitung 22 und die Diode 18 dem Regelverstärker 5 zugeführt. Solange zwischen der aktiven Elektrode 12 und dem Gewebe 9, die miteinander in Kontakt sind, kein elektrischer Lichtbogen zündet, wird die Ausgangsleistung des Leistungsverstärkers 4 vom Sollwertgeber 15 gesteuert. Sobald zwischen der aktiven Elektrode 12 und dem Gewebe 9 elektrische Lichtbogen 11 zünden und hierdurch nichtharmonische Frequenzen im Hochfrequenz-Ausgangskreis 13, 14 erscheinen, werden die für die Regelung der Intensität der elektrischen Lichtbogen 11 geeigneten nichtharmonischen Frequenzen durch das Filter 6 herausgefiltert, dem Gleichrichter 7 über die Leitung 25 zugeführt, dort gleichgerichtet und über die Leitung 24 dem Regelverstärker 5 zugeführt. Da das Ausgangssignal E des Gleichrichters 7 weitgehend proportional der Intensität der Lichtbogen 11 ist, wird die Ausgangsleistung des Leistungsverstärkers 4 durch das Ausgangssignal des Regelverstärkers mittels des Amplitudenmodulators 3 so gesteuert, daß die Intensität der Lichtbogen 11 auf den Sollwert des Sollwertgebers 15 geregelt wird.

## Patentansprüche

1. Hochfrequenz-Chirurgiegerät mit automatischer Regelung der Intensität der elektrischen Lichtbogen zwischen der aktiven Elektrode und dem zu schneidenden oder zu koagulierenden Gewebe, wobei zur Feststellung des Ausmaßes der Lichtbogen ein davon abhängiges elektrisches Signal über ein Filter abgeleitet und einem Gleichrichter (7) zugeführt wird, dessen Ausgangssignal (E) einem Regelverstärker (5) zugeführt wird, in welchem diese Ausgangssignal (E) entweder mit dem Sollwertsignal (S) eines Sollwertgebers (15) für den Schneidevorgang oder mit dem Sollwert (K) eines Sollwertgebers (8) für den Koagulationsvorgang verglichen wird, und wobei der Regelverstärker (5) ein der Differenz der Signale E minus S bzw. E minus K proportionales Signal (C) erzeugt, welches einem Amplitudenmodulator (3) zugeführt wird, welcher die Amplitude der Ausgangsspannung (U) des Leistungsverstärkers (4) steuert, **dadurch gekennzeichnet, daß** das Filter (6) derart ausgebildet ist, daß es mindestens eine Frequenz der von den elektrischen Lichtbogen (11) zwischen aktiver Elektrode (12) und Gewebe (9) erzeugten nichtharmonischen Frequenzen der Grundfrequenz ($f_1$) des Hochfrequenz-Oszillators (2) im Ausgang (13,14) durchläßt und gleichzeitig sowohl die Grundfrequenz ($f_1$) als auch deren harmonische Frequenzen so stark dämpft, daß sie keinen Einfluß auf das Regelsignal (C) haben, wobei die mindestens eine Frequenz aus der elektrischen Spannung (U) oder aus dem elektrischen Strom (I) zwischen aktiver Elektrode und Gewebe ermittelt wird.

2. Hochfrequenz-Chirurgiegerät nach Anspruch 1, **dadurch gekennzeichnet, daß** die untere Grenzfrequenz $f_g$ der Bandbreite $f_g - f_h$ des Filters (6) von der Harmonischen $f_n$ und die obere Grenzfrequenz $f_h$ der Bandbreite dieses Filters von der Harmonischen $f_{n+1}$ so viel Abstand $f_g$ minus $f_n$ bzw. $f_{n+1}$ minus $f_h$ haben, daß Seitenfrequenzen und Toleranzen der Frequenzen der Harmonischen $f_n$ und $f_{n+1}$ nicht in die Bandbreite $f_g - f_h$ des Filters (6) hineingeraten.

3. Hochfrequenz-Chirurgiegerät nach einem der vorhergehenden Ansprüche, **dadurch gekennzeichnet, daß** das Filter (6) ein piezoelektrisches Keramikfilter ist.

## Claims

1. High-frequency surgical apparatus with automatic control of the intensity of the electric arcs between the active electrode and the tissue to be out or to be coagulated, wherein, for establishing the size of the arc, an electrical signal dependent thereon is derived via a filter and is fed to a rectifier (7), the output signal (E) from which is fed to a control amplifier (5), in which this output signal (E) is compared either with the set-point signal (S) of a set-point transmitter (15) for the cutting operation or with the set-point (K) of a set-point transmitter (8) for the coagulation operation, and wherein the control amplifier (5) produces a signal (C), proportional to the difference of the signals E minus S or E minus K respectively, which signal (C) is supplied to an amplitude modulator (3), which controls the amplitude of the output voltage (U) of the power amplifier (4), characterized in that the filter (6) is so constructed that it passes at least one frequency of the non-harmonic frequencies of the basic frequency ($f_1$) of the high-frequency oscillator (2) generated by the electric arc (11) between active electrode (12) and tissue (9) in the output (13, 14) and simultaneously sufficiently damps the basic frequency ($f_1$) and also it harmonic frequencies that they do not have any influence upon the control signal (C), the at least one frequency being determined from the electrical voltage (U) or from the electrical current (1) between active electrode and tissue.

2. High-frequency surgical apparatus according to claim 1, characterized in that the lower limiting frequency $f_9$ of the band width $f_g - f_h$ of the filter (6) is at such a distance $f_g - f_n$ from the harmonic $f_n$, and the upper limiting frequency $f_h$ of the band width of this filter is at such a distance $f_n+1 - f_h$ from the harmonic $f_n+1$ that side frequencies and tolerances of the frequencies of the harmonics $f_n$ and $f_n+1$ do not enter into the band width $f_g - f_h$ of the filter (6).

3. High-frequency surgical apparatus according to one of the preceding claims, characterized in that the filter (6) is a piezo-electric ceramic filter.

## Revendications

1. Appareil chirurgical à haute fréquence à réglage automatique de l'intensité de l'arc électrique entre l'électrode active et le tissu à couper ou à coaguler, dans lequel pour déterminer l'importance des arcs, un signal électrique dépendant de ceux-ci est dérivé par l'intermédiaire d'un filtre, et envoyé à un redresseur (7) dont le signal de sortie (E) est envoyé à un amplificateur de réglage (5) dans lequel ce signal de sortie (E) est comparé soit au signal de valeur de consigne (S) d'un générateur de valeur de consigne (K) d'un générateur de valeur de consigne (8) pour l'opération de coagulation, et l'amplificateur de réglage (5) produisant un signal (C) proportionnel à la différence des signaux (E) moins (S) ou (E) moins (K), lequel signal (C) est envoyé à un modulateur d'amplitude (3) qui commande l'amplitude de la tension de sortie (U) de l'amplificateur de puissance (4), caractérisé en ce que le filtre (6) est conçu de manière à laisser passer à la sortie (13, 14) au moins une fréquence parmi les fréquences non harmoniques de la fréquence de base ($f_1$) de l'oscillateur à haute fréquence (2), produites par les arcs électriques (11) entre l'électrode active (12) et le tissu (9), et atténue en même temps la fréquence de base ($f_1$) ainsi que ses fréquences harmoniques, suffisamment fortement pour qu'elles n'aient aucune influence sur le signal de réglage (C), une fréquence au moins de la tension électrique (U) ou du courant électrique (I) étant déterminée entre l'électrode active et le tissu.

2. Appareil chirurgical à haute fréquence selon la revendication 1, caractérisé en ce que la fréquence de base $f_g$ inférieure de la largeur de bande $f_g - f_h$ du filtre (6) est à une distance $f_g - f_h$ de l'harmonique $f_n$, et la fréquence limite supérieure $f_h$ de la largeur de bande de ce filtre est à une distance $f_n+1 - f_h$ de l'harmonique $f_n+1$, distances suffisantes pour que les fréquences latérales et les tolérances des fréquences des harmoniques $f_n$ et $f_n+1$ ne tombent pas dans la largeur de bande $f_g - f_h$ du filtre (6).

3. Appareil chirurgical à haute fréquence selon l'une des revendications précédentes, caractérisé en ce que le filtre (6) est une filtre céramique piézo-électrique.

Fig.1

Fig.2

Fig.3

$$\frac{U_a}{U_e}$$

$U_e$

$U_a$

$f_n$ $f_g$ $f_h$ $f_{n+1}$

f

6

Fig.4

Fig.5